# EUROPÄISCHE PATENTSCHRIFT

(11) **EP 2 088 999 B1**
(45) Veröffentlichungstag und Bekanntmachung des Hinweises auf die Patenterteilung: **28.04.2010**
(21) Anmeldenummer: 07725423.3
(22) Anmeldetag: 22.05.2007
(51) Int. Cl.: A61K 9/00, A61K 9/10, A61K 9/20, A61K 31/00, A61K 33/00, A61K 36/23

(54) **PHARMAZEUTISCHE ZUSAMMENETZUNG ENTHALTEND EINEN MAGENSÄURE BINDENDEN UND/ODER NEUTRALISIERENDEN WIRKSTOFF WIE EIN ALGINSÄUREDERIVAT**
PHARMACEUTICAL COMPOSITION COMPRISING AN ACTIVE INGREDIENT WHICH NEUTRALIZES AND/OR BINDS GASTRIC ACID, SUCH AS AN ALGINIC ACID DERIVATIVE
COMPOSITION PHARMACEUTIQUE COMPRENANT UN AGENT LIANT ET/OU NEUTRALISANT L'ACIDE GASTRIQUE ET UN DÉRIVÉ D'ACIDE ALGINIQUE

(30) Priorität: 08.08.2006 DE 102006037298
(43) Veröffentlichungstag der Anmeldung: 19.08.2009
(73) Patentinhaber: Maria Clementine Martin Klosterfrau Vertriebsgesellschaft mbH, 50670 Köln (DE)
(72) Erfinder: VESTWEBER, Anna-Maria, 51399 Burscheid (DE); SCHLÜTER, Andreas, 29439 Lüchow (DE)
(74) Vertreter: Gesthuysen, von Rohr & Eggert
(86) Internationale Anmeldenummer: PCT/EP2007/004522
(87) Internationale Veröffentlichungsnummer: WO 2008/017337

(56) Entgegenhaltungen:
- EP-A- 0 484 106
- WO-A-94/12180
- WO-A-95/01795
- FR-A1- 2 563 108
- FR-A1- 2 636 532
- GB-A- 2 009 597
- US-A- 5 360 793
- US-A1- 2002 198 165
- TYTGAT G N ET AL: "CLINICAL AND LABORATORY STUDIES OF THE ANTACID AND RAFT-FORMING PROPERTIES OF RENNIE ALGINATE SUSPENSION" ALIMENTARY PHARMACOLOGY & THERAPEUTICS, BLACKWELL SCIENTIFIC PUBLICATIONS LTD., CAMBRIDGE, GB, Bd. 23, Nr. 6, 15. März 2006 (2006-03-15), Seiten 759-765, XP009073597 ISSN: 0269-2813
- HILL-E-A., WADE-G.: "An Investigation into the Use of Creep Viscosimetry for In Vitro Characterisation of the Raft Forming Properties of Tablets Containing Alginic Acid and Antacid Components" DRUG DEVELOPMENT AND INDUSTRIAL PHARMACY, Bd. 19, Nr. 15, 1993, Seiten 1931-1937, XP009088752

## Beschreibung

Die vorliegende Erfindung betrifft eine Zusammensetzung (d. h. eine pharmazeutische Zusammensetzung bzw. Zubereitung), insbesondere ein Antazidum, vorzugsweise in fester Dosierungsform (z. B. als Pulver oder als Tablette) oder in flüssiger Dosierungsform (z. B. als Suspension), welche - in jeweils wirksamen bzw. pharmazeutisch wirksamen Mengen - eine Kombination von mindestens einem Magensäure bindenden und/oder neutralisierenden Wirkstoff in Form von Magnesiumtrisilikat einerseits zusammen mit Alginsäure und/oder Alginsäuresalzen sowie gegebenenfalls mindestens einem pflanzlichen und/oder ätherischen Öl andererseits umfaßt.

Weiterhin betrifft die vorliegende Erfindung die Verwendung der erfindungsgemäßen Zusammensetzung bzw. der erfindungsgemäßen pharmazeutischen Zusammensetzung, insbesondere des erfindungsgemäßen Antazidums, zur prophylaktischen und/oder kurativen, insbesondere symptomatischen Behandlung von Hyperazidität des Magens - synomym bisweilen auch als "Hyperchlorhydrie" oder "Magen(saft)übersäuerung/-überproduktion" bezeichnet - und damit in Zusammenhang stehenden Folgeerscheinungen und Folgeerkrankungen, wie beispielsweise Sodbrennen, Ösophagitis (z. B. Refluxösophagitis), dyspeptischen Beschwerden, Reizmagen, Gastritis, Ulcuserkrankungen und dergleichen.

Magensäure ist eine sehr starke Säure. Die Magensäure hat die physiologischen Aufgaben, die Speisen zu verdauen und Krankheitskeime aus der Nahrung unschädlich zu machen. Damit die Magensäure nicht das umliegende Körpergewebe verdaut und schädigt, ist die Magenwand mit einer Schutzschicht ausgekleidet. Die Öffnungen zur Speiseröhre und zum Zwölffingerdarm sind mit Schließmuskeln abgedichtet. Diese Muskeln sorgen dafür, daß der Speisebrei von der Speiseröhre in den Magen und vom Magen in den Dünndarm fließt und nicht umgekehrt.

Die Magensäure wird in der Magenschleimhaut hauptsächlich in der Nacht produziert und dort auch gespeichert. Tagsüber wird sie dann - je nach Bedarf - aus den Speichern in den Magen abgegeben. Die Menge an Säure, die jeweils freigesetzt wird, hängt von vielen Faktoren ab. So wird besonders viel Magensäure bei reichhaltigem bzw. fettem Essen, aber auch durch Streß freigesetzt.

Entscheidend dabei ist aber das sinnvolle, physiologische Gleichgewicht zwischen aggressiven und defensiven Vorgängen im Magen; kommt es zu einem Ungleichgewicht, entstehen Magenprobleme von Sodbrennen bis hin zur Gastritis oder zum Magengeschwür.

Normalerweise funktioniert das zuvor geschilderte, ausgeklügelte System einwandfrei. Doch manchmal können Nahrungsmittel, Genußmittel, aber auch Krankheiten und Medikamente Beschwerden in Verbindung mit der Magensäure hervorrufen. Die Beschwerden bei Sodbrennen und Magenbeschwerden sind sehr ähnlich und oft nur schwer zu unterscheiden. Da die Magensäure durch Nahrung gebunden wird und dadurch an Aggressivität verliert, treten die Beschwerden meist erst einige Zeit, üblicherweise etwa eine bis zwei Stunden nach dem Essen auf oder aber nachts.

Eine sogenannte Hyperazidität des Magens kann unterschiedliche Ursachen haben: In vielen Fällen gibt es keine bekannten bzw. ermittelbaren Ursachen für die Neigung zu Sodbrennen und anderen, mit der Hyperazidität in Verbindung stehenden Folgeerscheinungen und Folgeerkrankungen; jedoch kann bisweilen einer der folgenden Gründe ausschlaggebend sein: So können übermäßiger Genuß von Kaffee, Alkohol und Nikotin, üppige, insbesondere fette Mahlzeiten, stark gewürzte und gebratene Speisen, Zitrusfrüchte, Fruchtsäfte, Zwiebeln, Schokolade, Tomaten etc. solche Beschwerden hervorrufen. Auch Streß und falsche Eßgewohnheiten (z, B. hastiges Hinunterschlingen von Speisen) können Ursache für eine Hyperazidität des Magens sein. Übergewichtige und Schwangere leiden häufig unter Sodbrennen, da die Größe und Körpermasse auf den Magen drückt. Auch Verstopfungen, Blähungen, eine erhöhte Magensäureproduktion oder eine verzögerte Magenentleerung können Ursachen für eine Hyperazidität des Magens sein. Auch können manche Medikamente als Nebenwirkung den Schließmuskel zur Speiseröhre erschlaffen lassen. Auch organische Ursachen, wie beispielsweise eine Erschlaffung des Schließmuskels, eine "verrutschte" Magenwand oder eine Hernie, können Ursache für eine Hyperazidität des Magens sein. Auch kann die Langzeiteinnahme von Schmerzmitteln aus der Gruppe der nichtsteroidalen Antiphlogistika, welche die Bildung der schützenden Prostaglandine hemmen, zu einer Hyperazidität des Magens fuhren.

Zunächst kommt es infolge der Hyperazidität zu Sodbrennen, verbunden mit einem brennenden Schmerz in der Brust, Wenn der Schließmuskel zwischen Magen- und Speiseröhre nicht optimal funktioniert fließt saurer Magenbrei in die Speiseröhre zurück und reizt dort die ungeschützte Schleimhaut, was zu einer sogenannten Refluxösophagitis führen kann. Gelangt der Magensaft sogar in den Mund, spricht man von saurem Aufstoßen. Sodbrennen tritt meist circa eine bis zwei Stunden nach den Mahlzeiten oder beim Bücken und Liegen auf.

Das Sodbrennen selbst ist keine Krankheit, sondern eine Beschwerde, welche mit unterschiedlichen Krankheiten zusammenhängen kann. Gelegentliches Sodbrennen ist unbedenklich. Tritt es jedoch mehrmals pro Woche auf, verätzt die Magensäure auf Dauer die empfindliche Schleimhaut der Speiseröhre. Die Folge kann eine Refluxösophagitis sein. Bei einer chronischen, also immer wiederkehrenden Speiseröhrenentzündung besteht die Gefahr, daß die betroffenen Schleimhautzellen entarten und sich ein Speiseröhrenkarzinom entwikkeln kann.

Eine leichte Reizung der Magenschleimhaut kann gleichermaßen eine Magenschleimhautentzündung (Gastritis) hervorrufen, welche im allgemeinen schnell abklingt. Bei Dauerreizung kann die Entzündung aber auch chronisch werden. Im Laufe der Zeit kann sich daraus eine Wunde in der Schleimhaut, insbesondere ein Magengeschwür, entwickeln. Auch hier besteht die Gefahr, daß die Schleimhautzellen entarten und sich zu einem Karzinom entwickeln.

Bei einem Reizmagen dagegen sind keine organischen Ursachen nachweisbar. Deshalb wird die Diagnose nur dann gestellt, wenn andere Erkrankungen ausgeschlossen werden können. Die Symptome sind sehr vielfältig: Manchmal stehen Sodbrennen und Aufstoßen im Vordergrund; andere Patienten leiden stärker unter Völlegefühl, Blähungen und Übelkeit.

Je nach Schwere und Ursache der Erkrankung kommen unterschiedliche Medikamente zum Einsatz: In leichteren Fällen genügt die Neutralisierung der Magensäure mit sogenannten Antazida, beispielsweise auf Basis von Hydroxiden, Carbonaten und Silikaten des Aluminiums, Magnesiums, Calciums und Natriums (z. B. Aluminiumhydroxid, Magnesiumhydroxid, Magnesiumtrisilikat, Calciumcarbonat, Natriumbicarbonat etc.). Während aluminiumhaltige Antazida den Nachteil einer Phosphatbindung beinhalten und oftmals zu Obstipationen führen und zudem in größeren Mengen zu Aluminium-Intoxikationen (z. B. Dialyse-Enzephalopathie) führen können und zudem andere Arzneimittel, wie Tetracycline, Digoxin, Chinolone etc., unerwünschterweise binden, können calciumbasierte Antazida zu einer sogenannten Hypercalcämie, zu einer Alkalose und in schweren Fällen auch zu Niereninsuffizienz (Milch-Alkali-Syndrom) führen. Natriumbicarbonat beispielsweise kann zu einer metabolischen Alkalose führten; auch ist die Natriumbelastung nicht zu vernachlässigen, und außerdem führt die CO₂-Bildung zu einem unerwünschten Aufstoßen und kann sogar zu einer Magenruptur führen. Zwar führen die vorgenannten Antazida zu einer Neutralisierung der Magensäure, jedoch wird kein zusätzlicher Schutz für das angegriffene Gewebe bereitgestellt.

In schweren Fällen kommen daher andere, wirksamere Medikamente zum Einsatz, beispielsweise Protonenpumpenblocker (z. B. Omeprazol, Lansoprazol, Pantoprazol, Esomeprazol etc.), Histamin-H₂-Rezeptoren-Antagonisten (z. B. Ranitidin, Famotidin, Nizatidin, Cimetidin etc.), Misoprostol (ein Prostaglandin-Derivat), Sucralfat und dergleichen. Diese Medikamente können jedoch zu schwerwiegenden Nebenwirkungen führen, die von Kopfschmerzen und Diarrhoe bis hin zu Myopathien, Gastrinerhöhung, Depressionen, Lethargie und Hepatitis reichen können.

Da bei Magengeschwüren fast immer das Bakterium Helicobacter pylori zu finden ist, muß hier zusätzlich eine Eradizierung mit geeigneten Antibiotika bzw. Antibiotikakombinationen durchgeführt werden.

Die EP 0 484 106 A1 betrifft eine Tablette zur kontrollierten Freisetzung einer biologisch aktiven Substanz, wobei die Tablette neben der biologisch aktiven Substanz ein wasserlösliches Alginat und ein Magnesium- oder Natriumantazidum aufweist, wobei das Verhältnis zwischen wasserlöslichem Alginat einerseits und Magnesium- bzw. Natriumantazidum andererseits 1 : 2 bis 50 : 1 betragen soll. Weiterhin ist es vorgesehen, der dort beschriebenen Zusammensetzung eine weitere biologisch aktive Substanz zuzugeben, wie Sedativa, Vasodilatatoren und dergleichen.

Weiterhin betrifft die GB 2 009 597 A ein Verfahren zur Herstellung einer gelförmigen oralen Dosierungseinheit mit antaziden Eigenschaften, welche eine gelartige Konsistenz aufweist, die durch Gelierung einer Dispersion eines Antazidums in Wasser mit formgebenden Mitteln, wie Kohlenhydraten oder mehrwertigen Alkoholen, erhalten wird. Die Dosiereinheit wird aus der gelierten Dispersion in Formen ausgeformt, Die Gelierungsmittel sollen dabei Gelatine, Agar, Nahrungsmittelstärke und Calciumalginat umfassen.

Des weiteren betrifft die WO 95/01795 A1 eine pharmazeutische Zusammensetzung zur Behandlung von gastrointestinalen Erkrankungen, wobei die Zusammensetzung neben Alginat und einem Antazidum einen pharmakologisch aktiven H₂-Antagonisten aufweist.

Die FR 2 563 108 A1 betrifft eine antazide pharmazeutische Zusammensetzung, welche Magnesiumalginat in Kombination mit einer zur Neutralisation von Säuren wirksamen Menge eines Antazidums enthält, wobei als Antazidum insbesondere Aluminiumhydroxid eingesetzt werden kann.

Weiterhin betrifft die Literaturstelle von Tytgat et al., "Clinical and laboratory studies of the antacid and raft-forming properties ofRennie alginate suspension", Alimentary Pharmacology & Therapeutics, Blackwell Scientific Publications Ltd., Cambridge, GB, Band 23, Nr. 6, 15. März 2006, Seiten 759 bis 765, XP009073597, ISSN: 0269-2813, eine Charakterisierung der antaziden und schwimmenden Eigenschaften einer Rennie^{®}-Alginatsuspension, wobei es sich hierbei um eine Suspension auf Basis von Calciumcarbonat und Magnesiumcarbonat handelt.

Des weiteren betrifft die WO 94/12180 A1 eine pharmazeutische Zusammensetzung, welche als H₂-Antagonisten Cimetidin enthält. Der Wirkstoff Cimetidin liegt in Form eines mit einem pflanzlichen Öl überzogenen Granulats vor. Des weiteren kann die Zusammensetzung ein Antazidum und ein Alginat aufweisen.

Weiterhin betrifft die US 2002/0198165 A1 ein Verfahren und eine Zusammensetzung zur Vorbeugung bzw. Behandlung von Magengeschwüren, wobei der zu behandelnden Person eine wirksame Menge einer Nukleinsäure zur Vorbeugung oder Behandlung des Magengeschwürs verabreicht werden soll.

Dabei kann die zur Verabreichung vorgesehene Zusammensetzung auch ein Antazidum sowie ein Alginat aufweisen.

Des weiteren betrifft die US 5 360 793 A eine antazide Zusammensetzung, welche in der Lage sein soll, eine schwimmende, gelatinöse Masse bei Kontakt mit wäßriger Säure zu bilden und welche neben Xanthangummi auch hexitolstabilisiertes Aluminiumhydroxid und ein gasbildendes Material aufweist, wobei das gasbildende Material bei Inkontaktbringen mit der wäßrigen Säure ein ungiftiges Gas produzieren soll.

Ferner betrifft die FR 2 636 532 A1 eine pharmazeutische Zusammensetzung zur Verwendung in der Human- oder Veterinärmedizin, welche neben Ranitidin auch Alginsäure und ein Carbonat oder Dicarbonat enthält. Die lediglich fakultative Verwendung von Magnesiumtrisilikat erfolgt ausschließlich in Kombination mit der zwingend vorgesehenen Verwendung eines Carbonates bzw. Dicarbonates als Antazidum.

Schließlich betrifft die Literaturstelle von Hill et al., "An Investigation Into the Use of Creep Viscometry for In Vitro Characterisation of the Raft Forming Properties of Tablets Containing Alginic Acid and Antacid Components", Drug Development and Industrial Pharmacy, 1993, 19(15), Seiten 1931 bis 1937, XP009088752, die schwimm- bzw. gleitbildenden Eigenschaften ("*Raft Forming Properties*") von Tabletten, welche neben Alginsäure auch antazide Wirksubstanzen enthalten.

Der vorliegenden Erfindung liegt die Aufgabe zugrunde, eine Zusammensetzung, insbesondere pharmazeutische Zubereitung, bereitzustellen, vorzugsweise in Form eines Antazidums, welche sich insbesondere zur prophylaktischen und/oder kurativen Behandlung von Hyperazidität des Magens und damit in Zusammenhang stehenden Folgeerscheinungen und Erkrankungen (z. B. Sodbrennen, Ösophagitis, wie z. B. Refluxösophagitis, dyspeptische Beschwerden, Reizmagen, Gastritis, Ulcuserkrankungen und dergleichen) eignet und die zuvor geschilderten Nachteile des Standes der Technik zumindest weitgehend vermeidet oder aber wenigstens abschwächt.

Die Anmelderin hat nun überraschenderweise herausgefunden, daß sich die zuvor geschilderte Aufgabe dadurch lösen läßt, daß man mindestens einen Magensäure bindenden und/oder neutralisierenden Wirkstoff in Form von Magnesiumtrisilikat mit Alginsäure und/oder mindestens einem physiologisch verträglichen Alginsäuresalz (Alginat) und gegebenenfalls mindestens einem pflanzlichen und/oder ätherischen Öl kombiniert.

Gegenstand der vorliegenden Erfindung- gemäß einem ersten Aspekt der vorliegenden Erfindung - ist somit eine Zusammensetzung, insbesondere pharmazeutische Zubereitung, welche sich insbesondere zur prophylaktischen und/oder kurativen Behandlung von Hyperazidität und damit in Zusammenhang stehenden Folgeerscheinungen und Erkrankungen eignet und vorzugsweise in Form eines Antazidums vorliegt, wobei die erfindungsgemäße Zusammensetzung - in Kombination und in jeweils wirksamen, insbesondere pharmazeutisch wirksamen Mengen -
(a) Magnesiumtrisilikat, in Mengen von 450 bis 550 mg pro Dosiereinheit, insbesondere je Tablette oder je Suspensionsbeutel;
(b) Alginsäure und/oder mindestens ein physiologisch verträgliches Alginsäuresalz in Mengen von insgesamt 300 bis 400 mg pro Dosiereinheit, insbesondere je Tablette oder je Suspensionsbeutel, wobei die Alginsäure und/oder das Alginsäuresalz eine relative Molmasse von 20.000 bis 250.000 Dalton aufweist; und
(c) gegebenenfalls mindestens ein pflanzliches und/oder ätherische Öl aus der Gruppe von Kümmelöl, Anisöl, Fenchelöl und Pfefferminzöl sowie deren Gemischen in Mengen von insgesamt 0,01 bis 20 mg pro Dosiereinheit, insbesondere je Tablette oder je Suspensionsbeutel,
enthält.

Der Begriff "pharmazeutische Zusammensetzung" bzw. "pharmazeutische Zubereitung", wie er im Rahmen der vorliegenden Erfindung verwendet wird, ist sehr breit zu verstehen und umfaßt nicht nur pharmazeutische Präparate bzw. Pharmazeutika und Arzneimittel als solche, sondern auch sogenannte Medizinprodukte.

Die Anmelderin hat überraschenderweise herausgefunden, daß durch die vorgenannte Kombination von Alginsäure bzw. mindestens eines physiologisch verträglichen Alginsäuresalzes (Alginats) zusammen mit mindestens einem Magensäure bindenden und/oder neutralisierenden Wirkstoff in Form von Magnesiumtrisilikat und gegebenenfalls mit mindestens einem pflanzlichen und/oder ätherischen Öl die zuvor geschilderte Aufgabenstellung in effizienter Weise gelöst und ein wirksames Therapeutikum für die Behandlung einer Hyperazidität des Magens und damit in Zusammenhang stehender Folgeerscheinungen und -erkrankungen bereitgestellt werden kann, welches nahezu nebenwirkungsfrei ist.

Die Alginsäure bzw. Alginate wird von Braunalgen in den Zellen gebildet und stellt in der Alge das strukturgebende Element dar. Die interzelluläre Gelmatrix verleitet der Alge sowohl Flexibilität als auch Festigkeit. Alginsäure ist unter anderem ein Nebenprodukt bei der Gewinnung von Iod aus Meeresalgen im Naßverfahren. Für die Verwendung in der Lebensmittel-, Pharma- und Kosmetikindustrie wird es gezielt aus Braunalgen extrahiert. Die Salze der Alginsäure werden im allgemeinen als Alginate bezeichnet. Alginate finden vor allem als Verdickungs- oder Geliermittel Verwendung. Alginsäure bzw. die Alginate sind Polysaccharide, welche aus 1,4-verknüpfter α-L-Guluronsäure (G) und β-D-Mannuronsäre (M) bestehen, wobei homopolymere Bereiche gebildet werden, in denen Mannuronsäure oder Guluronsäure als Block vorliegen, wobei diese Blöcke auch als GG- oder MM-Blöcke bezeichnet werden; im Bereich der GG- und MM-Blöcke kommt es zu einer Faltstruktur, die bei der Gelierung eine wesentliche Rolle spielt, insbesondere die GG-Blöcke bilden eine regelmäßige Zickzack-Struktur aus.

Im Rahmen der erfindungsgemäßen pharmazeutischen Zubereitung wirkt die Alginsäure bzw. das Alginat wie folgt: Bei Kontakt mit der Magensäure nimmt die Alginsäure bzw, das Alginat Wasser auf und bildet eine viskose Schicht. Im Magen beginnt die Alginsäure, eine viskose und schaumige Suspension zu bilden. Diese Schaumschranke aus Alginsäure bzw. Alginaten befindet sich auf dem spezifisch schwereren Mageninhalt und reduziert auf diese Weise rein physikalisch die Anzahl der gastroösophagialen Refluxepisoden. Wird der Mageninhalt kardiawärts gepreßt, schützt der Schaum aus Alginsäure bzw. Alginaten die Speiseröhre vor dem Kontakt mit der Magensäure. Der pH-Wert unterhalb der physikalischen Schaumschranke bleibt durch die Alginsäure selbst mit ca. 1 unangetastet, während die Schaumschranke selbst auf eine nahezu neutralen pH-Wert eingestellt wird - infolge der Anwesenheit des Magensäure neutralisierenden bzw, bindenden Wirkstoffs. Die auf dem Mageninhalt schwimmende Schaumschranke sinkt mit fortschreitender Entleerung des Magens ab und kommt am Pylorus in Kontakt mit alkalischen Valenzen des Duodenums. Bei ansteigendem pH-Wert löst sich die Schaumschranke auf und wird praktisch unverdaut ausgeschieden. Die normalen Verdauungsvorgänge bleiben unbeeinflußt, d. h. die Alginsäure bzw. die Alginate wirken rein physikalisch und werden nicht resorbiert. Der gleichzeitig vorhandene, Magensäure bindende bzw, neutralisierende Wirkstoff ist dagegen in der Lage, die überschüssige Magensäure zu binden bzw. zu neutralisieren. Zudem bewirkt er, daß die alginsäurehaltige physikalische Schaumschranke im wesentlichen pH-neutral ist.

Für die Zwecke der vorliegenden Erfindung wird eine Alginsäure bzw. ein Alginat mit einer relativen Molmasse von 20.000 bis 250.000 Dalton, insbesondere 30.000 bis 220.000 Dalton, besonders bevorzugt 40.000 bis 200.000 Dalton, eingesetzt.

Erfindungsgemäß wird also eine zuvor definierte Kombination von Alginsäure bzw. Alginaten mit Magnesiumtrisilikat und gegebenenfalls mit mindestens einem pflanzlichen und/oder ätherischen Öl eingesetzt, wie nachfolgend noch im Detail ausgeführt werden wird.

Wie die vorstehenden Ausführungen zeigen, ist durch die Kombination von Alginsäure bzw. Alginaten zusammen mit einem die Magensäure bindenden bzw. neutralisierenden Wirkstoff, nämlich Magnesiumtrisilikat, erstmals ein nahezu nebenwirkungsfreies Therapeutikum für die alleinige oder begleitende Behandlung von Hyperazidität des Magens und damit in Zusammenhang stehenden Folgeerscheinungen und -erkrankungen bereitgestellt worden, welches gleichzeitig die eingangs geschilderten Nachteile des Standes der Technik zumindest weitgehend vermeidet oder aber wenigstens abschwächt.

Neben den vorgenannten Inhaltsstoffen (a), (b) und gegebenenfalls (c) enthält die erfindungsgemäße Zusammensetzung üblicherweise zusätzlich mindestens einen pharmazeutischen Träger bzw. Exzipienten.

Im allgemeinen liegt die erfindungsgemäße Zusammensetzung in peroral verabreichbarer Applikationsform vor, Dabei kann die erfindungsgemäße Zusammensetzung entweder in fester oder aber in flüssiger Dosierungsform vorliegen.

Im Fall einer festen Dosierungsform liegt die erfindungsgemäße Zusammensetzung insbesondere als Pulver oder Tablette, vorzugsweise als Tablette, besonders bevorzugt als Kautablette, vor. Im Fall einer flüssigen Dosierungsform liegt die erfindungsgemäße Zusammensetzung insbesondere als Suspension (z. B. verpackt in einem dosierfertigen Suspensionsbeutel für die einmalige Anwendung) vor.

Die Menge an den jeweiligen Inhaltsstoffen in der erfindungsgemäßen Zusammensetzung kann in weiten Bereichen variieren. Im übrigen gilt, daß der Fachmann anwendungsbezogen oder einzelfallbedingt von den nachfolgend aufgeführten Mengenangaben abweichen kann, ohne daß er den Rahmen der vorliegenden Erfindung verläßt.

Wie zuvor beschrieben, enthält die erfindungsgemäße pharmazeutische Zusammensetzung den Magensäure bindenden und/oder neutralisierenden Wirkstoff (d. h. Magnesiumtrisilikat) per Dosiereinheit, insbesondere pro Tablette oder je Suspensionsbeutel, in Mengen von 450 bis 550 mg. Dennoch kann es im Einzelfall oder anwendungsbezogen erforderlich sein, von den vorgenannten Mengenangaben abzuweichen, ohne daß der Fachmann den Rahmen der vorliegenden Erfindung verläßt.

Was die Alginsäure und/oder das Alginsäuresalz anbelangt, so enthält die erfindungsgemäße pharmazeutische Zusammensetzung die Alginsäure und/oder das Alginsäuresalz pro Dosiereinheit, insbesondere je Tablette oder je Suspensionsbeutel, in Mengen von 300 bis 400 mg. Dennoch kann es im Einzelfall oder anwendungsbezogen erforderlich sein, von den vorgenannten Mengenangaben abzuweichen, ohne daß der Fachmann den Rahmen der vorliegenden Erfindung verläßt.

Was den erfindungsgemäß eingesetzten, Magensäure bindenden und/oder neutralisierenden Wirkstoff anbelangt, so kommen erfindungsgemäß Magnesiumtrisilikate (2 MgO - 3 SiO₂ bzw. 2 MgO· 3 SiO₂·x H₂O) zum Einsatz. Denn die Anmelderin hat überraschender Weise herausgefunden, daß sich mit Magnesiumtrisilikat - zusammen mit der Alginsäure bzw, den Alginaten - die besten Ergebnisse erhalten lassen, da Magnesiumtrisilikat nur langsam mit der Magensäure reagiert und diese physiologisch bindet und somit eine Langzeitwirkung entfaltet.

Magnesiumtrisilikat ist ein kristallines, geruchsloses weißes Pulver, welches praktisch unlöslich in Wasser und Alkohol ist. Oral eingenommene Magnesiumtrisilikat reagiert mit der Magensäure nur langsam und wir dabei selbst in Magnesiumchlorid und Siliciumdioxid umgewandelt, um dann als unlösliche Magnesiumverbindung über den Darm ausgeschieden zu werden. Auf diese Weise wirkt Magnesiumtrisilikat über einen verlängerten Zeitraum antazid und ist dabei in der Lage, überschüssige Magensäure langsam und damit physiologisch zu binden, um so den möglichen Säure-Rebound zu verhindern,

Diesbezüglich unterscheidet sich die Trisilikatverbindung deutlich von anderen antazid wirksamen Salzen, auch von anderen Magnesiumsalzen, besonders von Magnesiumcarbonat, und anderen Hydroxidverbindungen. In den erfindungsgemäß vorgesehen Mengen führt das Magnesiumtrisilikat auch dazu, daß ein weicher Stuhl erreicht wird. Im Unterschied zu Aluminiumhydroxidverbindungen können Obstipationen in wirksamer Weise verhindert werden. Jedoch sollte eine kritische Höchstgrenze nicht überschritten werden, da andernfalls Durchfall einsetzen kann.

Neben den beiden vorgenannten Wirkstoflkomponenten (a) und (b) kann die erfindungsgemäße pharmazeutische Zusammensetzung (c) mindestens ein pflanzliches und/oder ätherisches Öl enthalten. Das pflanzliche und/oder ätherische Öl ist insbesondere ausgewählt aus der Gruppe von Kümmelöl, Anisöl, Fenchelöl und Pfefferminzöl sowie deren Gemischen, Wie zuvor beschrieben, kann die erfindungsgemäße Zusammensetzung das oder die pflanzlichen und/oder ätherischen Öle in Mengen von insgesamt 0,01 bis 20 mg pro Dosiereinheit, insbesondere je Tablette oder je Suspensionsbeutel, enthalten.

Die fakultative pflanzliche und/oder ätherische Ölkomponente (c) fördert die Prokinetik bzw, wirkt prokinetisch, d. h. beeinflußt die Abläufe im Magen/Darm-Trakt positiv und kann spastische Kontraktionen im Magen/DarmTrakt verhindern und insbesondere spasmolytische Wirkung entfalten. So besitzt beispielsweise Kümmelöl spasmolytische Wirkungen an der glatten Muskulatur des Magen/Darm-Traktes sowie antimikrobielle Eigenschaften; diese begründen auch die karminativen Effekte bei dyspeptischen Beschwerden bei Völlegefühl und Blähungen. Fenchelöl wirkt an der glatten Muskulatur gleichermaßen spasmolytisch. Auch Anisöl hat spasmolytische und antibakterielle Wirkungen. Gleichermaßen besitzt auch das in den Pfefferminzblättern enthaltene ätherische Öl spasmolytische Wirkung in bezug auf die glatte Muskulatur des Magen/Darm-Traktes und wirkt darüber hinaus cholagog und karminativ. Darüber hinaus sind die vorgenannten pflanzlichen bzw. ätherischen Öle in bezug auf die Geschmacks- und Geruchseigenschaften der erfindungsgemäßen pharmazeutischen Zusammensetzung förderlich.

Wie zuvor beschrieben, betrifft somit die vorliegende Erfindung eine Zusammensetzung, insbesondere pharmazeutische Zusammensetzung, vorzugsweise in Form eines Antazidums, welche sich insbesondere zur prophylaktischen und/oder kurativen Behandlung von Hyperazidität des Magens und damit in Zusammenhang stehenden Folgeerscheinungen und Erkrankungen eignet, wobei die Zusammensetzung - in Kombination und in jeweils wirksamen, insbesondere pharmazeutisch wirksamen Mengen -
(a) Magnesiumtrisilikat in Mengen von 450 bis 550 mg pro Dosiereinheit, insbesondere je Tablette oder je Suspensionsbeutel;
(b) Alginsäure und/oder mindestens ein physiologisch verträgliches Alginsäuresalz in Mengen von insgesamt 300 bis 400 mg pro Dosiereinheit, insbesondere je Tablette oder je Suspensionsbeutel; und
(c) gegebenenfalls mindestens ein pflanzliches und/oder ätherisches Öl aus der Gruppe von Kümmelöl, Anisöl, Fenchelöl und Pfefferminzöl sowie deren Gemischen in Mengen von insgesamt 0,01 bis 20 mg pro Dosiereinheit, insbesondere je Tablette oder je Suspensionsbeutel,
enthält.

Neben den vorgenannten Wirk- und Inhaltsstoffen kann die erfindungsgemäße Zusammensetzung außerdem weitere Inhaltsstoffe enthalten. Diese können insbesondere ausgewählt sein aus Zusatz- und/oder Hilfsstoffen, wie beispielsweise Füll-, Streck-, Binde-, Netz-, Stabilisierungs-, Färbe-, Puffer-, Riech-, Geschmacks-, Süßungs-, Aroma-, Verarbeitungshilfs- und/oder Konservierungsstoffen und -mitteln,

Im allgemeinen liegt die erfindungsgemäße pharmazeutische Zusammensetzung, wie sie vorstehend beschrieben ist, in Dosiereinheiten, insbesondere Tabletten, Pulverbeuteln oder Suspensionsbeuteln, von 0,1 bis 5 g, insbesondere 0,5 bis 3 g, vorzugsweise 0,5 bis 2 g, vor. Diese Dosiereinheiten sind vorteile hafterweise nach dem Essen, insbesondere ein bis zwei Stunden nach dem Essen, und/oder vor dem Nachtschlaf einzunehmen, und zwar jeweils in Mengen von ein bis zwei Dosiereinheiten, und zwar in Abhängigkeit von der Schwere des Krankheitsbildes,

Die zuvor beschriebene pharmazeutische Zusammensetzung ist ein sicheres Therapeutikum zur prophylaktischen und/oder kurativen, insbesondere symptomatischen Behandlung von Hyperazidität des Magens und damit in Zusammenhang stehenden Folgeerscheinungen und Folgeerkrankungen der eingangs genannten Art. Die erfindungsgemäße Zusammensetzung führt zu keinen bzw, nahezu keinen systemischen Nebenwirkungen, gewährleistet aber dennoch eine sichere symptomatische Therapie der säurebedingten Magen- und Speiseröhrenerkrankungen - entweder als Monotherapeutikum oder begleitend zu anderen Therapeutika der eingangs genannten Art. Mit der Kombination von Alginsäure bzw, Alginaten mit Magnesiumtrisilikat wird eine besonders langanhaltende Wirkung erhalten.

Des weiteren betrifft die vorliegenden Erfindung - gemäß einem **zweiten** Aspekt der vorliegenden Erfindung - die Verwendung der erfindungsgemäßen pharmazeutischen Zusammensetzung. In bezug auf die erfindungsgemäß Verwendung kann - zur Vermeidung unnötiger Wiederholungen. - auf die vorstehenden Ausführungen zu der erfindungsgemäßen Zusammensetzung selbst verwiesen werden, welche in bezug auf die erfindungsgemäße Verwendung entsprechend gelten.

Insbesondere betrifft die vorliegende Erfindung die Verwendung der erfindungsgemäßen Zusammensetzung, wie zuvor beschrieben, als Antazidum.

Des weiteren betritt die vorliegende Erfindung die Verwendung der erfindungsgemäßen Zusammensetzung, wie zuvor beschrieben, zur prophylaktischen und/oder kurativen, insbesondere symptomatischen prophylaktischen und/oder kurativen Behandlung bzw. zur Herstellung eines Arzneimittels zur prophylaktischen und/oder kurativen, insbesondere symptomatischen prophylaktischen und/oder kurativen Behandlung von Hyperazidität (d. h. Hyperazidität des Magens) und damit in Zusammenhang stehenden Folgeerscheinungen und -erkrankungen.

Insbesondere betrifft die vorliegende Erfindung die Verwendung der zuvor beschriebenen erfindungsgemäßen Zusammensetzung zur prophylaktischen und/oder kurativen, insbesondere symptomatischen prophylaktischen und/oder kurativen Behandlung bzw, zur Herstellung eines Arzneimittels zur prophylaktischen und/oder kurativen, insbesondere symptomatischen prophylaktischen und/oder kurativen Behandlung von Sodbrennen, Ösophagitis (z. B. Refluxösophagitis), dyspeptischen Beschwerden, Reizmagen, Gastritis, Ulcuserkrankungen und dergleichen.

Bei der erfindungsgemäßen Verwendung wird die erfindungsgemäße Zusammensetzung üblicherweise nach einer Mahlzeit und/oder vor dem Nachtschlaf appliziert. Diesbezüglich kann auf die vorstehenden Ausführungen verwiesen werden.

Mit der erfindungsgemäßen Zusammensetzung wird erstmals ein nahezu nebenwirkungsfreies Therapeutikum zur prophylaktischen und/oder kurativen Behandlung von Hyperazidität des Magens und damit in Zusammenhang stehenden Folgeerscheinungen und -erkrankungen der eingangs genannten Art bereitgestellt, welches auf einer Kombination mindestens eines Magensäure bindenden und/oder neutralisierenden Wirkstoffs zusammen mit Alginsäure und/oder deren Salzen beruht und eine effiziente Langzeitwirkung entfaltet und auf diese Weise sowohl eine Monotherapie als auch eine begleitende Therapie der vorgenannten Erkrankungen ermöglicht.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Das nachfolgende Ausführungsbeispiel dient lediglich der Veranschaulichung der vorliegenden Erfindung, ohne sie jedoch hierauf zu beschränkten.

### Ausführungsbeispiel:

20 Patienten mit klinisch und endoskopisch diagnostizierter Refluxösophagitis wurden in vier Gruppen mit jeweils fünf Patienten unterteilt. Den einzelnen Patientengruppen wurden unterschiedliche Magentabletten verabreicht (Kautabletten zu jeweils 1 g mit den nachfolgend spezifizierten Wirkstoffmengen, Restmenge: Exzipient sowie übliche Hilfsstoffe). Patientengruppe I erhielt Magentabletten auf Basis von 350 mg Alginsäure und 500 mg Magnesiumtrisilikat (erfindungsgemäß), Patientengruppe II auf Basis von 350 mg Alginsäure und 500 mg Aluminiumhydroxid (ebenfalls erfindungsgemäß), Patientengruppe III auf Basis nur von 500 mg Magnesiumtrisilikat (nichterfindungsgemäß) und Patientengruppe IV nur auf Basis von 500 mg Alummiumhydroxid (gleichermaßen nichterfindungsgemäß). Die Therapie wurde jeweils über 6 Wochen durchgeführt, wobei jeweils zwei Tabletten ein bis zwei Stunden nach den Mahlzeiten verabreicht wurden sowie ein bis zwei Tabletten vor dem Nachtschlaf.

Bei allen Patientengruppen zeigte sich kurz nach Einnahme der jeweiligen Magentabletten eine Linderung von auftretendem Sodbrennen. Bei den Vergleichstherapiegruppen III und IV war der Effekt jedoch nicht langanhaltend (ca. ½ bis 1 Stunde), wohingegen er bei den erfindungsgemäßen Therapeutika der Patientengruppen I und II bis zur nächsten Mahlzeit andauerte. Im Fall der erfindungsgemäßen Magentabletten mit Magnesiumtrisilikat wurde ein noch länger anhaltender Effekt als bei den erfindungsgemäßen Magentabletten mit Aluminiumhydroxid beobachtet.

Nach bereits 3 Wochen war im Fall der Patientengruppe I in allen fünf Fällen die Refluxösophagitis vollständig austherapiert, wurde jedoch die Therapie zur Verhinderung von Rezidiven noch weitere 3 Wochen fortgesetzt. Bei der erfindungsgemäßen Therapie der Patientengruppe II dauerte die Behandlung 1½ Wochen länger; auch in diesem Fall wurde die Therapie um weitere 3 Wochen fortgesetzt, um Rezidive zu verhindern. Bei den Vergleichsprodukten konnte auch nach 5 Wochen noch keine Ausheilung der Refluxösophagitis beobachtet werden; dort wurde die Therapie weitere 4 Wochen mit dem erfindungsgemäßen Präparat auf Basis von Alginsäure und Magnesiumtrisilikat fortgeführt, wobei dann in 3 Wochen auch in diesen Patientengruppen eine vollständige Ausheilung der Refluxösophagitis eintrat und die Therapie nachfolgend 3 weitere Wochen fortgesetzt wurde, um Rezidive zu verhindern.

Während bei den erfindungsgemäßen Magentabletten auf Basis von Alginsäure und Magnesiumtrisilikat keine Nebenwirkungen auftraten, wurden bei den erfindungsgemäßen Magentabletten auf Basis von Alginsäure und Aluminiumhydroxid in vier von fünf Fällen Obstipationen beobachtet, die in drei der vier Fälle mit einem Laxativum auf pflanzlicher Basis behandelt werden mußten.

Alle Diagnosen und Anamnesen wurden klinisch wie endoskopisch ermittelt und verfolgt.

Die vorangehende Patientenstudie belegt eindrucksvoll die Überlegenheit der erfindungsgemäßen binären Wirkstoffkombination aus Magensäure neutralisierendem bzw. bindendem Wirkstoff einerseits und Alginsäure andererseits, wobei durch die Verwendung einer magnesiumhaltigen, Magensäure neutralisierenden bzw. bindenden Wirksubstanz (Magnesiumtrisilikat) die Langzeitwirkung gegenüber einer analogen aluminiumhaltigen Wirksubstanz (Aluminiumhydroxid) noch gesteigert werden kann und Nebenwirkungen, insbesondere Obstipationen, in wirksamer Weise verhindert werden können.

## Patentansprüche

1. Zusammensetzung, insbesondere zur Verwendung zur prophylaktischen und/oder kurativen Behandlung von Hyperazidität, wobei die Zusammensetzung - in Kombination und in jeweils wirksamen, insbesondere pharmazeutisch wirksamen Mengen -
(a) Magnesiumtrisilikat, in Mengen von 450 bis 550 mg pro Dosiereinheit, insbesondere je Tablette oder je Suspensionsbeutel;
(b) Alginsäure und/oder mindestens ein physiologisch verträgliches Alginsäuresalz in Mengen von insgesamt 300 bis 400 mg pro Dosiereinheit, insbesondere je Tablette oder je Suspensionsbeutel, wobei die Alginsäure und/oder das Alginsäuresalz eine relative Molmasse von 20.000 bis 250.000 Dalton aufweist; und
(c) gegebenenfalls mindestens ein pflanzliches und/oder ätherisches Öl aus der Gruppe von Kümmelöl, Anisöl, Fenchelöl und Pfefferminzöl sowie deren Gemischen in Mengen von insgesamt 0,01 bis 20 mg pro Dosiereinheit, insbesondere je Tablette oder je Suspensionsbeutel,
enthält.

2. Zusammensetzung nach Anspruch 1, enthaltend außerdem einen pharmazeutischen Träger.

3. Zusammensetzung nach Anspruch 1 oder 2, wobei die Zusammensetzung in peroral verabreichbarer Applikationsform vorliegt.

4. Zusammensetzung nach einem oder mehreren der vorangehenden Ansprüche, wobei die Zusammensetzung in fester oder flüssiger Dosierungsform vorliegt.

5. Zusammensetzung nach einem oder mehreren der vorangehenden Ansprüche, wobei die Alginsäure und/oder das Alginsäuresalz eine relative Molmasse von 30.000 bis 220.000 Dalton, insbesondere 40.000 bis 200.000 Dalton, aufweist.

6. Zusammensetzung nach einem oder mehreren der vorangehenden Ansprüche, wobei die Zusammensetzung außerdem weitere Inhaltsstoffe enthält, insbesondere ausgewählt aus Zusatz- und/oder Hilfsstoffen, wie Füll-, Streck-, Binde-, Netz-, Stabilisierungs-, Färbe-, Puffer-, Riech-, Geschmacks-, Süßungs-, Aroma-, Verarbeitungshilfs- und/oder Konservierungsstoffen und -mitteln.

7. Zusammensetzung nach einem oder mehreren der vorangehenden Ansprüche, wobei die Zusammensetzung in Dosiereinheiten, insbesondere Tabletten, Pulverbeuteln oder Suspensionsbeuteln, von 0,1 bis 5 g, insbesondere 0,5 bis 3 g, vorzugsweise 0,5 bis 2 g, vorliegt.

8. Verwendung einer Zusammensetzung nach den vorangehenden Ansprüchen als Antazidum.

9. Verwendung einer Zusammensetzung nach den vorangehenden Ansprüchen zur prophylaktischen und/oder kurativen, insbesondere symptomatischen prophylaktischen und/oder kurativen Behandlung bzw, zur Herstellung eines Arzneimittels zur prophylaktischen und/oder kurativen, insbesondere symptomatischen prophylaktischen und/oder kurativen Behandlung von Hyperazidität und damit in Zusammenhang stehenden Folgeerscheinungen und Erkrankungen eingesetzt wird.

10. Verwendung einer Zusammensetzung nach den vorangehenden Ansprüchen zur prophylaktischen und/oder kurativen, insbesondere symptomatischen prophylaktischen und/oder kurativen Behandlung bzw. zur Herstellung eines Arzneimittels zur prophylaktischen und/oder kurativen, insbesondere symptomatischen prophylaktischen und/oder kurativen Behandlung von Sodbrennen, Ösophagitis, insbesondere Refluxösophagitis, dyspeptischen Beschwerden, Reizmagen, Gastritis, Ulcuserkrankungen und dergleichen.

11. Verwendung nach einem oder mehreren der vorangehenden Ansprüche, wobei die Zusammensetzung nach einer Mahlzeit appliziert wird.

## Claims

1. Composition, more particularly for use for prophylactic and/or curative treatment of hyperacidity, the composition comprising - in combination and in respectively effective, more particularly pharmaceutically effective amounts -
(a) magnesium trisilicate, in amounts of 450 to 550 mg per dosage unit, more particularly per tablet or per suspension bag;
(b) alginic acid and/or at least one physiologically tolerable alginic acid salt in amounts of altogether 300 to 400 mg per dosage unit, more particularly per tablet or per suspension bag, the alginic acid and/or the alginic acid salt having a relative molar mass of 20,000 to 250,000 daltons; and
(c) optionally at least one vegetable and/or essential oil from the group consisting of caraway oil, anise oil, fennel oil and peppermint oil and also their mixtures in amounts of altogether 0.01 to 20 mg per dosage unit, more particularly per tablet or per suspension bag.

2. Composition according to Claim 1, further comprising a pharmaceutical carrier.

3. Composition according to Claim 1 or 2 in an oral administration form.

4. Composition according to one or more of the preceding claims in a solid or liquid dosage form.

5. Composition according to one or more of the preceding claims, wherein the alginic acid and/or the alginic acid salt has a relative molar mass of 30,000 to 220,000 daltons, more particularly 40,000 to 200,000 daltons.

6. Composition according to one or more of the preceding claims, additionally comprising further ingredients, more particularly selected from added and/or auxiliary substances, such as filling, extending, binding, wetting, stabilizing, colouring, buffering, scent, taste, sweetening, aroma, processing aid and/or preservative substances and agents.

7. Composition according to one or more of the preceding claims in dosage units, more particularly tablets, powder bags or suspension bags, of 0.1 to 5 g, more particularly 0,5 to 3 g, preferably 0.5 to 2 g.

8. Use of a composition according to the preceding claims as an antacid.

9. Use of a composition according to the preceding claims for prophylactic and/or curative, more particularly symptomatic prophylactic and/or curative treatment, or in the manufacture of a pharmaceutical for prophylactic and/or curative, more particularly symptomatic prophylactic and/or curative treatment of hyperacidity and related sequelae and disorders.

10. Use of a composition according to the preceding claims for prophylactic and/or curative, more particularly symptomatic prophylactic and/or curative treatment, or in the manufacture of a pharmaceutical for prophylactic and/or curative, more particularly symptomatic prophylactic and/or curative treatment of heartburn, oesophagitus, more particularly reflux oesophagitis, dyspeptic conditions, irritable stomach, gastritis, ulcers and the like.

11. Use according to one or more of the preceding claims, wherein the composition is administered after a meal.

## Revendications

1. Composition, en particulier destinée à une utilisation pour le traitement prophylactique et/ou curatif de l'hyperacidité, où la composition contient - en combinaison et à chaque fois à des quantités actives, en particulier pharmaceutiquement actives -
(a) du trisilicate de magnésium, en des quantités de 450 à 550 mg par unité de dosage, en particulier par comprimé ou par sachet de suspension;
(b) de l'acide alginique et/ou au moins un sel d'acide alginique physiologiquement acceptable en des quantités au total de 300 à 400 mg par unité de dosage, en particulier par comprimé ou par sachet de suspension, où l'acide alginique et/ou le sel d'acide alginique présente une masse molaire relative de 20.000 à 250.000 Daltons; et
(c) le cas échéant au moins une huile végétale et/ou essentielle du groupe formé par l'huile de cumin, l'huile d'anis, l'huile de fenouil et l'huile de menthe poivrée ainsi que leurs mélanges en des quantités au total de 0,01 à 20 mg par unité de dosage, en particulier par comprimé ou par sachet de suspension.

2. Composition selon la revendication 1, contenant en outre un support pharmaceutique.

3. Composition selon la revendication 1 ou 2, où la composition se trouve sous une forme d'administration par voie perorale.

4. Composition selon l'une ou plusieurs des revendications précédentes, où la composition se trouve sous une forme de dosage solide ou liquide.

5. Composition selon l'une ou plusieurs des revendications précédentes, où l'acide alginique et/ou le sel d'acide alginique présente une masse molaire relative de 30.000 à 220.000 Daltons, en particulier de 40.000 à 200.000 Daltons.

6. Composition selon l'une ou plusieurs des revendications précédentes, où la composition contient en outre d'autres constituants, en particulier choisis parmi les additifs et/ou les adjuvants, tels que les substances et agents de charge, diluant(e)s, liant(e)s, mouillant(e)s, de stabilisation, colorant(e)s, tampon, odoriférant(e)s, gustatives/gustatifs, édulcorant(e)s, de parfum, de transformation et/ou de conservation.

7. Composition selon l'une ou plusieurs des revendications précédentes, où la composition se trouve dans des unités de dosage, en particulier des comprimés, des sachets de poudre ou de suspension, de 0,1 à 5 g, en particulier de 0,5 à 3 g, de préférence de 0,5 à 2 g.

8. Utilisation d'une composition selon les revendications précédentes comme antiacide.

9. Utilisation d'une composition selon les revendications précédentes pour le traitement prophylactique et/ou curatif, en particulier prophylactique et/ou curatif symptomatique ou pour la préparation d'un médicament destiné au traitement prophylactique et/ou curatif, en particulier prophylactique et/ou curatif symptomatique de l'hyperacidité et des conséquences et maladies qui y sont associées.

10. Utilisation d'une composition selon les revendications précédentes pour le traitement prophylactique et/ou curatif, en particulier prophylactique et/ou curatif symptomatique ou pour la préparation d'un médicament destiné au traitement prophylactique et/ou curatif, en particulier prophylactique et/ou curatif symptomatique des aigreurs d'estomac, de l'oesophagite, en particulier de l'oesophagite de reflux, des troubles dyspepsiques, d'un estomac irritable, de la gastrite, des maladies ulcératives et analogues.

11. Utilisation selon l'une ou plusieurs des revendications précédentes, où la composition est administrée après un repas.
